# DEMANDE DE BREVET EUROPEEN

(11) **EP 0 755 906 A1**
(43) Date de publication de la demande: **29.01.1997**
(21) Numéro de dépôt: 96401532.5
(22) Date de dépôt: 11.07.1996
(51) Int. Cl.: C07C 2/62

(54) **Procédé d'alyklation catalytique d'hydrocarbures aliphatiques**

(30) Priorité: 20.07.1995 FR 9508931
(71) Demandeur: INSTITUT FRANCAIS DU PETROLE, 92502 Rueil-Malmaison (FR)
(72) Inventeur: Benazzi, Eric, 78360 Montesson (FR); Joly, Jean-François, 69006 Lyon (FR); Ferrer, Nathalie, 78360 Montesson (FR); Torck, Bernard, 92100 Boulogne sur Seine (FR)

(57) **Abrégé**

L'invention concerne l'utilisation en alkylation catalytique d'au moins une isoparaffine choisie dans le groupe formé par l'isobutane et l'isopentane en présence d'au moins une oléfine comportant de 3 à 6 atomes de carbone par molécule d'un catalyseur comprenant entre 40 et 99% poids de l'acide choisi parmi les acides de formule R-SO₃H, où R est le fluor ou un groupement alkyl ou un groupement alkyl fluoré, R étant de préférence F ou CF₃, de façon encore plus préférée CF₃, et entre 1 et 60% poids d'un solvant choisi dans le groupe formé par le sulfolane, le diméthylsulfoxyde et les dioxannes, de préférence le sulfolane.

## Description

La présente invention concerne un catalyseur comprenant entre 40 et 99% poids de l'acide choisi parmi les acides de formule R-SO₃H, où R est le fluor ou un groupement alkyl ou un groupement alkyl fluoré, R étant de préférence F ou CF₃, de façon encore plus préférée CF₃, et entre 1 et 60% poids d'un solvant choisi dans le groupe formé par le sulfolane, les dioxannes et le diméthylsulfoxyde de préférence le sulfolane et plus particulièrement son utilisation en alkylation dite aliphatique, c'est-à-dire en alkylation catalytique d'isoparaffine C₄-C₅, c'est-à-dire comportant 4 ou 5 atomes de carbone par molécule (isobutane et/ou isopentane), au moyen d'au moins une oléfine comprenant de 3 à 6 atomes de carbone par molécule, (c'est-à-dire d'une oléfine C₃-C₆), ce qui permet d'obtenir des composés paraffiniques à haut degré de ramification et à haut indice d'octane.

Un grand nombre de catalyseurs acides, liquides ou solides, sont connus pour réaliser l'alkylation aliphatique d'isoparaffine(s) telles que l'isobutane ou l'isopentane, par au moins une oléfine telle que le propylène, les butènes-1 et -2, l'isobutène. Les catalyseurs les plus couramment utilisés dans la pratique industrielle sont les catalyseurs liquides que sont l'acide sulfurique concentré et l'acide fluorhydrique seul ou en mélange avec des acides de Lewis tels que le trifluorure de bore. Ces procédés souffrent d'inconvénients majeurs : l'acide fluorhydrique en raison de sa toxicité et de sa forte volatilité; l'acide sulfurique en raison d'une consommation importante du catalyseur nécessitant un retraitement onéreux.

Il a été proposé dans le brevet US-A-3.795.712 d'utiliser, pour catalyser les réactions d'alkylation de composés aromatiques par au moins une oléfine ou les réactions d'alkylation aliphatique d'isobutane par au moins un oléfine ou les réactions d'oligomérisation ou de polymérisation d'oléfines, des compositions catalytiques comprenant un acide de Lewis ou de Brönsted et une sulfone de formule R-SO₂-R', où R et R' sont chacun séparément un radical monovalent comportant de 1 à 8 atomes de cabone par molécule, ou forment ensemble un radical divalent comportant de 3 à 12 atomes de carbone, éventuellement dans un solvant hydrocarboné inerte, la concentration de l'acide étant comprise entre 10⁻⁵ moles par litre de sulfone et 5 moles par litre de sulfone, ce qui fait entre 0,00012 et 37% poids d'acide dans le cas où l'acide est l'acide trifluorométhane sulfonique et la sulfone est le sulfolane.

La demande de brevet PCT WO 93/00316 décrit des compositions catalytiques pour l'alkylation aliphatique comprenant de 10 à 90% poids d'acide fluorhydrique ou sulfonique substitué par au moins un halogène et de 10 à 90% poids d'un solvant possédant un nombre donneur inférieur à 40 en l'absence d'ajout intentionnel d'halogénure de métal. Parmi de tels solvants dont la liste est nombreuse figurent le sulfolane ou tétraméthylène sulfone et le diméthylsulfoxyde.

Le catalyseur utilisé selon la présente invention constitue un perfectionnement des compositions catalytiques décrites dans US-A-3.795.712 et WO 93/00316, utilisé en alkylation aliphatique.

La présente invention concerne l'utilisation en alkylation d'isoparaffine C₄-C₅ par au moins une oléfine C₃-C₆ d'un catalyseur comprenant entre 40 et 99% poids d'un acide choisi parmi les acides de formule R-SO₃H, où R est le fluor ou un groupement alkyl ou un groupement alkyl fluoré (partiellement ou totalement fluoré), R étant de préférence F ou CF₃, de façon encore plus préférée CF3, et entre 1 et 60% poids d'un solvant choisi dans le groupe formé par le sulfolane, nom déposé du bioxyde de tétrahydrothiofène, le diméthylsulfoxyde (encore appelé diméthylsulfinone) et les dioxannes (le dioxanne 1-2 et le dioxanne 1-4), de préférence le sulfolane. De préférence le catalyseur comprend donc de l'acide fluorosulfonique HSO3H ou trifluorométhanesulfonique CF₃SO₃H et du solvant. De façon encore plus préférée, le catalyseur comprend de l'acide trifluorométhanesulfonique et du solvant. Ledit catalyseur comprend (en % poids) entre 40 et 99%, de préférence entre 45 et 98% et de manière encore plus préférée entre 55 et 97% poids d'acide et entre 1 et 60%, de préférence entre 2 et 55% et de manière encore plus préférée entre 3 et 45% poids dudit solvant.

L'acide utilisé possède généralement un titre, en % poids, compris entre 95 et 100% et de préférence entre 98 % et 100 % poids, le complément à 100 % étant le plus souvent constitué d'eau.

Le solvant utilisé est généralement miscible à l'acide et non miscible à la phase hydrocarbonée. La basicité de ce solvant doit être la plus faible possible de manière à ne pas diminuer l'activité protonique de l'acide avec lequel il est en mélange.

Le catalyseur utilisé selon l'invention est donc un catalyseur en phase liquide.

Un des avantages du catalyseur utilisé selon l'invention, par rapport à l'acide sulfurique de titre 97 à 99% poids actuellement utilisé dans les unités d'alkylation, est qu'il présente une acidité supérieure ou égale et un caractère oxydant beaucoup plus faible, compte tenu du fait que l'acide compris dans ledit catalyseur possède un caractère oxydant beaucoup plus faible que l'acide suflurique. D'autre part, un des avantages du catalyseur utilisé selon l'invention, par rapport à l'acide trifluorométhanesulfonique, est la limitation du passage indésirable en solution hydrocarbonée de l'acide compris dans ledit catalyseur, sous forme de composés dénommés triflates d'alkyle dans le cas où l'acide est l'acide trifluorométhanesulfonique.

Un des avantages du catalyseur utilisé selon l'invention est aussi la qualité de l'alkylat obtenu, qui présente une teneur en soufre moindre que lors de l'utilisation d'acide sulfurique ou d'acide trifluorométhanesulfonique.

Un autre des avantages du catalyseur utilisé selon la présente invention est que l'utilisation dudit catalyseur conduit donc à une réduction de la consommation de catalyseur et donc à une diminution des coûts opératoires des unités d'alkylation.

Le procédé de préparation du catalyseur utilisé selon l'invention comprend généralement au moins deux étapes. La première étape consiste en une purification du solvant, par exemple par distillation, puis dans une deuxième étape l'acide et le solvant sont mélangés dans les proportions désirées.

L'invention concerne ainsi l'utilisation dudit catalyseur en alkylation catalytique d'au moins une isoparaffine choisie dans le groupe formé par l'isobutane et l'isopentane (c'est-à-dire l'isobutane et/ou l'isopentane: l'isobutane, ou l'isopentane, ou l'isobutane et l'isopentane) en présence d'au moins une oléfine comportant de 3 à 6 atomes de carbone par molécule.

Le catalyseur utilisé selon la présente invention est mis en oeuvre dans un procédé qui permet de réaliser dans les meilleures conditions la réaction d'alkylation de l'isoparaffine par au moins une oléfine. En particulier, ladite réaction étant caractérisée par une forte exothermicité (environ 83,6 kJ/mol de butène transformé si l'oléfine est le butène et si l'isoparaffine est l'isobutane), la mise en oeuvre du catalyseur utilisé selon la présente invention permet d'obtenir une bonne homogénéité de température et de concentration en réactifs. En particulier, le catalyseur selon la présente invention est mis en oeuvre par toutes les techniques connues de l'homme du métier et dont le dispositif de mise en oeuvre comprend au moins un réacteur et au moins un décanteur.

Les procédés existant pour la production d'hydrocarbures par alkylation de l'isobutane par les oléfines utilisent généralement des procédés où le milieu réactionnel est biphasique (soit l'acide sulfurique soit l'acide fluorhydrique pour les procédés commerciaux existants). La phase continue est soit la phase acide soit la phase hydrocarbonée. En général, la phase continue est la phase acide ; le catalyseur acide constitue une phase liquide qui est mise en contact avec le mélange isobutane-oléfine(s) liquide pour former une émulsion et le rapport volumique acide/hydrocarbures est supérieur à 1. Par exemple, dans le cas de la mise en oeuvre de l'acide sulfurique par la technologie Stratco (L.F. Albright, Chem. Eng., August 15, 1966, p. 143 et L.F. Albright, Oil & Gas Journal, November 12, 1990), qui est la plus répandue, l'émulsion est créée, à une extrémité d'un réacteur horizontal, par une turbine alimentée par la charge et le recyclage d'acide. Mais une mise en oeuvre où la phase continue est la phase hydrocarbonée est aussi envisageable (voir par exemple la demande de brevet PCT W095/04.019).

Les procédés utilisés selon la présente invention sont généralement des procédés où le milieu réactionnel est biphasique, comportant une phase acide et une phase hydrocarbonée. La phase continue est soit la phase acide soit la phase hydrocarbonée.

La température de réaction est généralement inférieure à +20° C, de préférence inférieure à +15° C et de manière souvent plus préférée comprise entre +5° C et -5° C. La pression de la zone réactionnelle est suffisante pour maintenir les hydrocarbures à l'état liquide dans ladite zone.

La vitesse spatiale horaire, exprimée en poids d'oléfine introduite par unité de poids du catalyseur et par heure (pph) comprise entre 0,001 et 10 h⁻¹ et de préférence comprise entre 0,002 et 2 h⁻¹. Dans tous les cas, le mélange constitué en zone réactionnelle est dans des conditions de pression et de température telles que le mélange d'hydrocarbures reste liquide.

Afin de limiter les réactions secondaires, on peut utiliser un excès d'isoparaffine(s) par rapport à l' (les) oléfine(s). A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butène(s) dans la charge est généralement compris entre 1 et 100, de préférence entre 3 et 50 et de manière souvent préférée entre 5 et 15.

Lorsque la nature du catalyseur et les conditions de réaction sont judicieusement choisies (en particulier la température), le catalyseur selon l'invention permet la production de produits d'alkylation d'au moins une isoparaffine par au moins une oléfine qui sont intéressants comme carburants pour les moteurs et constituants d'essence et qui comprennent par exemple au moins 60 % moles de paraffines possédant 8 atomes de carbone par molécule et moins de 1 % moles de composés non saturés, les paraffines comprenant 8 atomes de carbone par molécule comprenant 70 à 98 % en moles de triméthylpentanes.

Les exemples suivants illustrent l'invention sans toutefois en limiter la portée.

### Exemple 1 : Préparation du catalyseur 1 conforme à l'invention

On prépare 100 g de phase acide conforme à l'invention en mélangeant 80 g d'acide trifluorométhanesulfonique de titre 98 % poids et 20 g de sulfolane préalablement distillé sous vide. La phase acide ainsi préparée contient donc 20 % poids de sulfolane et 80 % poids d'acide trifluorométhanesulfonique de titre 98 % poids.

### Alkylation de l'isobutane par le butène-2 avec le catalyseur 1 en phase continue hydrocarbonée

On introduit 25 g du catalyseur 1 préparé selon la méthode décrite ci-dessus dans un réacteur en verre d'un volume de 500 ml préalablement purgé sous débit d'argon. Le réacteur contenant le catalyseur est alors fermé puis il est refroidi à la température de -1°C.

Ensuite, on introduit dans le réacteur 100 ml d'isobutane, puis le milieu réactionnel est agité avec une vitesse de rotation de 2000 tours par minute. Ledit réacteur est refroidi à -1 °C par circulation d'un liquide plus froid dans la double enveloppe dont il est équipé. L'agitation est obtenue par l'intermédiaire d'un axe de rotation équipé d'une turbine.

Après 30 minutes d'homogénéisation, on ajoute régulièrement 190 g d'un mélange constitué de 10 % poids de butène-2 et de 90 % poids d'isobutane, pendant une durée totale de 5 heures, la température du réacteur étant maintenue à -1° C pendant toute la durée de l'injection.

Après réaction et décantation la phase hydrocarbonée est soutirée du réacteur, isolée puis analysé par chromatographie en phase vapeur ; sa composition pondérale est donnée dans le tableau 1.

### Exemple 2 : Préparation du catalyseur 2 non conforme à l'invention

Le catalyseur utilisé dans le présent exemple est l'acide trifluorométhanesulfonique de titre 98 % poids sans ajout de solvant.

### Alkylation de l'isobutane par le butène-2 avec le catalyseur 2 en phase continue hydrocarbonée

On répète le test catalytique d'alkylation de l'isobutane par le butène-2 dans les mêmes conditions expérimentales que celles décrites dans l'exemple 1, et les mêmes analyses sont réalisées. La seule différence est que le présent exemple on introduit dans le réacteur 20 g d'acide. Les résultats sont donnés dans le tableau 1.

### Exemple 3 : Préparation du catalyseur 3 conforme à l'invention

On prépare 100 g de phase acide conforme à l'invention en mélangeant 80 g d'acide trifluorométhanesulfonique de titre 100% poids et 20 g de sulfolane préalablement distillé sous vide. Les 80 g d'acide trifluorométhanesulfonique de titre 100 % poids sont prélevés d'une préparation de 100 g d'acide trifluorométhanesulfonique obtenue par mélange de 76,13 g d'acide trifluorométhanesulfonique de titre 98 % poids et de 23,87 g de l'anhydride de l'acide trifluorométhanesulfonique (CF₃SO₂)₂O. La phase acide ainsi préparée contient donc 20 % poids de sulfolane et 80 % poids d'acide trifluorométhanesulfonique (CF₃SO₃H) de titre 100 % poids.

### Alkylation de l'isobutane par le butène-2 avec le catalyseur 3 en phase continue hydrocarbonée

On introduit 25 g du catalyseur 3 préparé selon la méthode décrite ci-dessus dans un réacteur en verre d'un volume de 500 ml préalablement purgé sous débit d'argon. Le réacteur contenant le catalyseur est alors fermé puis il est refroidi à la température de -1°C.

Ensuite, on introduit dans le réacteur 100 ml d'isobutane, puis le milieu réactionnel est agité avec une vitesse de rotation de 2000 tours par minute. Ledit réacteur est refroidi à -1° C par circulation d'un liquide plus froid dans la double enveloppe dont il est équipé. L'agitation est obtenue par l'intermédiaire d'un axe de rotation équipé d'une turbine.

Après 30 minutes d'homogénéisation, on ajoute régulièrement 190 g d'un mélange constitué de 10 % poids de butène-2 et de 90 % poids d'isobutane, pendant une durée totale de 5 heures, la température du réacteur étant maintenue à -1° C pendant toute la durée de l'injection.

Après réaction et décantation, la phase hydrocarbonée est soutirée du réacteur, isolée puis analysée par chromatographie en phase vapeur ; sa composition pondérale est donnée dans le tableau 1.

### Exemple 4 : Préparation du catalyseur 4 non conforme à l'invention

On prépare 100 g de phase acide conforme à l'invention en mélangeant 10 g d'acide trifluorométhanesulfornique de titre 100% poids et 90 g de sulfolane préalablement distillé sous vide. Les 10 g d'acide trifluorométhanesulfonique de titre 100 % poids sont prélevés d'une préparation de 100 g d'acide trifluorométhanesulfonique de titre 100 % poids obtenue par mélange de 76,13 g d'acide trifluorométhanesulfonique de titre 98 % poids et de 23,87 g de l'anhydride de l'acide trifluorométhanesulfonique (CF₃SO₂)₂O. La phase acide ainsi préparée contient donc 90 % poids de sulfolane et 10 % poids d'acide trifluorométhanesulfonique (CF₃SO₃H) de titre 100 % poids.

### Alkylation de l'isobutane par le butène-2 avec le catalyseur 4 en phase continue hydrocarbonée

On introduit 25 g du catalyseur 4 préparé selon la méthode décrite ci-dessus dans un réacteur en verre d'un volume de 500 ml préalablement purgé sous débit d'argon. Le réacteur contenant le catalyseur est alors fermé puis il est refroidi à la température de -1° C.

Ensuite, on introduit dans le réacteur 100 ml d'isobutane, puis le milieu réactionnel est agité avec une vitesse de rotation de 2000 tours par minute. Ledit réacteur est refroidi à -1° C par circulation d'un liquide plus froid dans la double enveloppe dont il est équipé. L'agitation est obtenue par l'intermédiaire d'un axe de rotation équipé d'une turbine.

Après 30 minutes d'homogénéisation, on ajoute régulièrement 190 g d'un mélange constitué de 10 % poids de butène-2 et de 90 % poids d'isobutane, pendant une durée totale de 5 heures, la température du réacteur étant maintenue à -1° C pendant toute la durée de l'injection.

Après réaction et décantation, la phase hydrocarbonée est soutirée du réacteur, isolée puis analysée par chromatographie en phase vapeur ; sa composition pondérale est donnée dans le tableau 1.

Il est à noter que dans cet exemple, contrairement aux exemples précédents, la conversion du butène-2 n'est pas complète et est d'environ 75%.

**Tableau 1 :**

| **Comparaison des catalyseurs 1, 2, 3 et 4** | | | | |
|---|---|---|---|---|
| Composition alkylat (% poids) | Exemple 1, catalyseur 1 conforme à l'invention | Exemple 2, catalyseur 2 non conforme à l'invention | Exemple 3, catalyseur 3 conforme à l'invention | Exemple 4, catalyseur 4 non conforme à l'invention |
| C₅-C₇ | 2,3 | 1,5 | 2,6 | 6,7 |
| C₈ | 92,6 | 84,2 | 93,7 | 57,7 |
| C₉⁺ | 5,1 | 14,3 | 3,7 | 35,6 |

Ce tableau met en évidence l'intérêt qu'il y a à travailler avec des catalyseurs selon l'invention, c'est à dire comprenant une phase acide comportant de l'acide trifluorométhanesulfonique et un solvant aprotique organique faiblement basique. En effet, les catalyseurs selon l'invention conduisent à de meilleures sélectivités que celui non conforme à l'invention.

D'autre part, l'utilisation du catalyseur 2 non conforme à l'invention conduit à un alkylat fumant, c'est-à-dire contenant de l'acide trifluorométhanesulfonique CF₃SO₃H, et par conséquent ledit alkylat est beaucoup plus chargé en triflates d'alkyle que les alkylats obtenus avec les catalyseurs 1 et 3 qui ne présentent pas une telle caractéristique. Dans le cas du catalyseur 4 non conforme à l'invention, la non conformité du catalyseur conduit à conversion incomplète de l'oléfine et surtout à une très mauvaise qualité de l'alkylat (trop de lourds C₉⁺),

### Exemple 5 : Préparation du catalyseur 5 non conforme à l'invention

On prépare 100 g de phase acide non conforme à l'invention en mélangeant 80 g d'acide trifluorométhane sulfonique de titre 100 % poids et 20 g de triéthylamine, dont le nombre donneur est égal à 30,5 (proche du nombre donneur du diméthylsulfoxyde égal à 29,8).

### Alkylation de l'isobutane par le butène-2 avec le catalyseur 5 en phase continue hydrocarbonée

On introduit 25 g du catalyseur 5 préparé selon la méthode décrite ci-dessus dans un réacteur en verre d'un volume de 500 ml préalablement purgé sous débit d'argon. Le réacteur contenant le catalyseur est alors fermé puis il est refroidi à la température de -1° C.

Ensuite, on introduit dans le réacteur 100 ml d'isobutane, puis le milieu réactionnel est agité avec une vitesse de rotation de 2000 tours par minute. Ledit réacteur est refroidi à -1° C par circulation d'un liquide plus froid dans la double enveloppe dont il est équipé. L'agitation est obtenue par l'intermédiaire d'un axe de rotation équipé d'une turbine.

Après 30 minutes d'homogénéisation, on ajoute régulièrement 190 g d'un mélange constitué de 10 % poids de butène-2 et de 90 % poids en volume d'isobutane, pendant une durée totale de 5 heures, la température du réacteur étant maintenue à -1° C pendant toute la durée de l'injection.

La conversion du butène-2 est très faible, ce qui est certainement dû à une acidité insuffisante du solvant.

### Exemple 6 : Préparation du catalyseur 6 non conforme à l'invention

On prépare 100 g de phase acide non conforme à l'invention en mélangeant 80 g d'acide trifluorométhane sulfonique de titre 100 % poids et 20 g d'acétone, dont le nombre donneur est égal à 17,0 (proche du nombre donneur du sulfolane égal à 14,8). On obtient un mélange coloré rouge signe d'une réaction entre l'acétone et l'acide trifluorométhane sulfonique.

Dans ces conditions un test d'alkylation réalisé comme décrit dans les exemples précédents ne conduit pas à la formation d'alkylat.

## Revendications

1. Utilisation en alkylation catalytique d'au moins une isoparaffine choisie dans le groupe formé par l'isobutane et l'isopentane en présence d'au moins une oléfine comportant de 3 à 6 atomes de carbone par molécule d'un catalyseur comprenant entre 40 et 99 % poids de l'acide choisi parmi les acides de formule R-SO₃H, où R est le fluor ou un groupement alkyl ou un groupement alkyl fluoré, et, entre 10 et 60% poids d'un solvant choisi dans le groupe formé par le sulfolane, le diméthylsulfoxyde et les dioxannes.

2. Utilisation selon la revendication 1 dans laquelle l'acide possède généralement un titre compris entre 95 et 100% poids.

3. Utilisation selon l'une des revendications 1 ou 2 dans laquelle le solvant est le sulfolane.

4. Utilisation selon l'une des revendications 1 à 3 dans laquelle l'acide est l'acide fluorosulfonique ou l'acide trifluorométhane sulfonique.

5. Utilisation selon l'une des revendications 1 à 4 dans laquelle l'acide est l'acide triifluorométhane sulfonique.

6. Utilisation selon l'une des revendications 1 à 5 dans laquelle la température de réaction est inférieure à +20° C, la pression de la zone réactionnelle est suffisante pour maintenir les hydrocarbures à l'état liquide dans ladite zone et le milieu réactionnel est biphasique.

7. Utilisation selon la revendication 6 telle que la phase continue est la phase acide.

8. Utilisation selon la revendication 6 telle que la phase continue est la phase hydrocarbonée.
